(19) Europäisches Patentamt / European Patent Office / Office européen des brevets

(11) **EP 3 916 104 A1**

(12) **EUROPEAN PATENT APPLICATION**
published in accordance with Art. 153(4) EPC

(43) Date of publication:
**01.12.2021 Bulletin 2021/48**

(51) Int Cl.:
*C12Q 1/02* (2006.01)   *C12M 1/34* (2006.01)
*G01N 33/53* (2006.01)   *G01N 33/566* (2006.01)

(21) Application number: **19911323.4**

(22) Date of filing: **06.11.2019**

(86) International application number:
**PCT/JP2019/043560**

(87) International publication number:
**WO 2020/152940 (30.07.2020 Gazette 2020/31)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **25.01.2019 JP 2019011069**

(71) Applicant: **Hamamatsu Photonics K.K.**
**Hamamatsu-shi, Shizuoka 435-8558 (JP)**

(72) Inventors:
• **KIMURA Yuji**
 **Hamamatsu-shi, Shizuoka 435-8558 (JP)**
• **KAZAMI Sayaka**
 **Hamamatsu-shi, Shizuoka 435-8558 (JP)**
• **HASHIMOTO Yu**
 **Hamamatsu-shi, Shizuoka 435-8558 (JP)**
• **ITOH Hiroyasu**
 **Hamamatsu-shi, Shizuoka 435-8558 (JP)**

(74) Representative: **Grünecker Patent- und Rechtsanwälte**
**PartG mbB**
**Leopoldstraße 4**
**80802 München (DE)**

(54) **METHOD FOR SELECTING CELLS HIGHLY RESPONSIVE TO TARGET SUBSTANCE, AND METHOD FOR DETERMINING CONCENTRATION OF TARGET SUBSTANCE WITH UNKNOWN CONCENTRATION IN SPECIMEN**

(57)   A method for selecting cells highly responsive to a target substance according to the present invention comprises step of: (1a) bringing a sample containing a target substance into contact with a plurality of cells, wherein the cells are cells having a receptor for the target substance and a fluorescent indicator, and the fluorescent indicator emits fluorescence as a result of binding between the target substance and the receptor; (1b) calculating a fluorescence intensity increase rate for each of the cells; and (1c) selecting arbitrary cells exhibiting a fluorescence intensity increase rate within the top 50% among the plurality of cells. According to the present invention, it is possible to detect the target substance in the sample with high sensitivity.

*Fig.5*

**Description**

**Technical Field**

The present invention relates to a method for selecting cells highly responsive to a target substance and a method for determining a concentration of a target substance with an unknown concentration in a sample.

**Background Art**

Conventionally, a method for detecting an odor substance using a mechanism by which an insect identifies an odor is known. For example, Patent Literature 1 discloses a method for detecting an odor substance in a sample by bringing a sample into contact with *Spodoptera frugiperda* cells that co-express an olfactory receptor protein of an insect and fluorescent protein. In such a method, a sample containing an odor substance is brought into contact with a container holding a cell chip, an average brightness value of a fluorescent image of the container before the odor substance is brought into contact is compared with an average brightness value of a fluorescent image of the container after the odor substance is brought into contact, and when the rate of increase of the average brightness value is a predetermined value or larger, it is determined that an odor substance has been detected.

**Citation List**

**Patent Literature**

[Patent Literature 1] Japanese Unexamined Patent Publication No. 2013-27376

**Summary of Invention**

**Technical Problem**

In the method described in Patent Literature 1, the detection sensitivity may not be sufficient. Therefore, an object of the present invention is to detect a target substance in a sample with high sensitivity.

**Solution to Problem**

A method for selecting cells highly responsive to a target substance according to the present invention comprises steps of: (1a) bringing a sample containing a target substance into contact with a plurality of cells, wherein the cells are cells having a receptor for the target substance and a fluorescent indicator, and the fluorescent indicator emits fluorescence as a result of binding between the target substance and the receptor; (1b) calculating a fluorescence intensity increase rate for each of the cells; and (1c) selecting arbitrary cells exhibiting a fluorescence intensity increase rate within the top 50% among the plurality of cells.

The above method may be performed using a microchannel device. The microchannel device comprises a first channel, a second channel adjacent to the first channel, and a communicating portion that connects the first channel to the second channel, and has an opening on the side of the first channel in which the cells can be captured. Step 1a may comprise: (A1) introducing a suspension containing the plurality of cells into the first channel so that a pressure in the first channel is higher than a pressure in the second channel to capture the cells in the opening on the side of the first channel; and (A2) introducing a sample containing a target substance into the first channel or the second channel while maintaining a pressure difference between the first channel and the second channel to bring the sample containing a target substance into contact with the captured cells. The method may comprise, after step 1c, steps of: (1d) introducing a liquid into the second channel so that the pressure in the second channel is higher than the pressure in the first channel to release the captured cells from the opening; and (1e) collecting the selected arbitrary cells among the released cells.

In step 1c, arbitrary cells exhibiting a fluorescence intensity increase rate within the top 30% may be selected among the plurality of cells.

A method for determining a concentration of a target substance with an unknown concentration in a sample according to the present invention comprises steps of: (2a) bringing a standard sample containing a target substance with a known concentration into contact with a plurality of cells, wherein the cells are cells having a receptor for the target substance and a fluorescent indicator, and the fluorescent indicator emits fluorescence as a result of binding between the target substance and the receptor; (2b) calculating a fluorescence intensity increase rate for each of the cells; (2c) repeating a combination of step 2a and step 2b using one or more standard samples containing the target substance with different concentrations to calculate a fluorescence intensity increase rate for each standard sample; (2d) bringing a sample containing a target substance with an unknown concentration into contact with the cells; (2e) calculating a fluorescence intensity increase rate for each of the cells after the sample is brought into contact; (2f) selecting arbitrary cells in which the fluorescence intensity increase rate calculated for the sample or any of the standard samples is within the top 50% among the cells; and (2g) comparing the fluorescence intensity increase rate calculated for the sample in step 2e with the fluorescence intensity increase rate calculated for the standard samples in step 2b and step 2c to calculate a concentration of the target substance in the sample, wherein the compared increase rates are the increase rates calculated for the cells selected in step 2f, wherein steps 2a, 2b, 2c, 2d, 2e, and 2g are performed in this order, and step 2f is performed at any stage after step 2b and before step 2g.

The above method may be performed using a microchannel device. The microchannel device comprises a first channel, a second channel adjacent to the first channel, and a communicating portion that connects the first channel to the second channel and has an opening on the side of the first channel in which the cells can be captured. The method may comprise, before step 2a, a step of introducing a suspension containing the plurality of cells into the first channel so that a pressure in the first channel is higher than a pressure in the second channel to capture the cells in the opening on the side of the first channel. Step 2a may comprise introducing the standard sample into the first channel or the second channel while maintaining a pressure difference between the first channel and the second channel to bring the standard sample into contact with the captured cells. Step 2d may comprise introducing the sample into the first channel or the second channel while maintaining a pressure difference between the first channel and the second channel, and bringing the sample into contact with the captured cells.

In step 2f, arbitrary cells in which the fluorescence intensity increase rate calculated for the sample or any of the standard samples is within the top 30% may be selected among the above cells.

In any of the above methods, the fluorescence intensity increase rate may be a time rate of change of "It" over a predetermined time. "It" is (Ft-F0)/F0. F0 represents a fluorescence intensity of the cells before the sample or the standard sample is brought into contact, and Ft represents a fluorescence intensity of the cells at any stage after the sample or the standard sample is brought into contact and before the fluorescence intensity reaches a plateau. The predetermined time is a time between any two stages after the sample or the standard sample is brought into contact and before the fluorescence intensity reaches a plateau. The fluorescence intensity increase rate may be a maximum value of a time rate of change of "It".

A method for determining a concentration of a target substance with an unknown concentration in a sample according to another aspect of the present invention comprises steps of: (3a) bringing a standard sample containing a target substance with a known concentration into contact with a plurality of cells, wherein the cells are cells having a receptor for the target substance and a fluorescent indicator, and the fluorescent indicator emits fluorescence as a result of binding between the target substance and the receptor; (3b) calculating a fluorescence intensity increase rate for each of the cells; (3c) repeating a combination of step 3a and step 3b using one or more standard samples containing the target substance with different concentrations to calculate a fluorescence intensity increase rate for each standard sample; (3d) bringing a sample containing a target substance with an unknown concentration into contact with the cells; (3e) calculating a fluorescence intensity increase rate for each of the cells after the sample is brought into contact; and (3f) comparing the fluorescence intensity increase rate calculated for the sample in step 3e with the fluorescence intensity increase rate calculated for the standard samples in step 3b and step 3c to calculate a concentration of the target substance in the sample, in this order.

In any of the above methods, the target substance may be an odor substance, and the plurality of cells may be insect cells having an olfactory receptor of an insect and a calcium-sensitive fluorescent protein. In any of the above methods, the target substance may be bombykal (hereinafter abbreviated as BAL), and the plurality of cells may be Sf21 cells having BmOR-3 protein and GCaMP6s protein.

### Advantageous Effects of Invention

By using the cells selected by the method according to the present invention, it is possible to detect a target substance in a sample with high sensitivity. In addition, according to the method for determining a concentration of a target substance with an unknown concentration in a sample according to the present invention, even if the concentration of the target substance is low, it is possible to determine the concentration.

### Brief Description of Drawings

FIG. 1 is a schematic view of a microchannel device that may be used to select highly responsive cells and to determine a concentration of a target substance.
FIG. 2(A) and FIG.2(B) are schematic views of a microchannel device that may be used to select highly responsive cells and to determine a concentration of a target substance.

FIG. 3 is a graph showing change in It over time.

FIG. 4(A), FIG. 4(B), and FIG. 4(C) show histograms of a distribution of a maximum increase rate of a fluorescence intensity. FIGS. 4(A), 4(B), and 4(C) show the results obtained when 30000 nM, 3000 nM, and 300 nM BAL solutions, respectively, were used.

FIG. 5 is a graph showing average values of values within the top 100%, 30%, 25%, and 20% in the distribution in FIGS. 4(A) to 4(C).

**Description of Embodiments**

A method for selecting cells highly responsive to a target substance according to the present invention comprises steps of:

(1a) bringing a sample containing a target substance into contact with a plurality of cells;
(1b) calculating a fluorescence intensity increase rate for each of the cells; and
(1c) selecting arbitrary cells exhibiting a fluorescence intensity increase rate within the top 50% among the plurality of cells.

Cells used in the present invention are cells having a receptor for a target substance and a fluorescent indicator.

The target substance is not particularly limited, and may be an odor substance such as BAL, bombykol, 1-octen-3-ol, geosmin, phenethyl alcohol, methyl benzoate, ethyl benzoate, benzyl alcohol, methyl salicylate, benzaldehyde, pentanal, hexanal, E2-hexanal, 2-heptanone, 6-methyl-5-hepten-2-one, and 2-methylphenol.

The Receptor for a target substance is not particularly limited, and may be, for example, a receptor for an odor substance. The Receptor for an odor substance may be, for example, an olfactory receptor of an insect such as silkworm moth, *Drosophila melanogaster,* and *Anopheles gambiae.* Examples of an olfactory receptor of silkworm moth include BmOR-3 protein which is a receptor for BAL and BmOR-1 protein which is a receptor for bombykol. Examples of an olfactory receptor of *Drosophila melanogaster* include Or13a protein which is a receptor for 1-octen-3-ol and Or56a protein which is a receptor for geosmin.

The fluorescent indicator is not particularly limited as long as it is a substance that emits fluorescence as a result of binding between the target substance and the receptor. The fluorescent indicator may be, for example, a fluorescent protein or a fluorescent dye. The fluorescent indicator is preferably a genetically encoded fluorescent protein. For example, when the binding between the target substance and the receptor results in a change of the ion concentration in the cells, the fluorescent indicator may be a fluorescent protein or fluorescent dye that is sensitive to the ion. Examples of the fluorescent protein include GCaMP3 protein, GCaMP6s protein, and GCaMP7 protein which are calcium-sensitive fluorescent proteins. Examples of the fluorescent dye include calcium-sensitive fluorescent dyes such as Fluo 3-AM, Rhod 2-AM, Calbryte (trademark) 520, Fluo 4-AM, Fura 2-AM, Indo 1-AM, Calbryte 590, and Calbryte 630.

Cells may further contain a co-receptor. An Example of a co-receptor is Orco protein which is a co-receptor for an insect olfactory receptor.

Cells may be, for example, cells derived from *Spodoptera frugiperda.* Examples of cells derived from *Spodoptera frugiperda* include Sf21 and Sf9.

In step 1a, a sample containing a target substance is brought into contact with a plurality of cells. By bringing the sample into contact with the cells, the target substance in the sample binds to the receptor, and as a result, the fluorescent indicator emits fluorescence. For example, when the receptor is a calcium ion channel such as a BmOR-3 protein, the binding of the target substance to the receptor causes influx of calcium ions into cells. Calcium ions bind to a calcium-sensitive fluorescent indicator present in cells, and the fluorescent indicator emits fluorescence. By continuing to bring the sample into contact with the cells, the concentration of calcium in the cells increases and the intensity of the fluorescence emitted from the cells also increases. However, the speed of influx of calcium ions into the cells gradually decreases and the concentration of calcium in the cells reaches a plateau (constant), and thus, the fluorescence intensity also reaches a plateau.

A method for bringing the sample into contact with the cells is not particularly limited, and when the sample is a liquid, for example, cells may be immersed in the sample for a predetermined time, or the sample may be caused to flow at a predetermined flow rate and cells may be placed in the flow. When the sample is a gas, for example, cells may be placed in a space filled with the sample. The concentration of the sample in step 1a may be unknown or known.

In step 1b, the fluorescence intensity increase rate is calculated for each cell. The fluorescence intensity may be a measured value itself (absolute value) or a relative value with respect to the fluorescence intensity of the cells before the sample is brought into contact. The fluorescence intensity increase rate may be a time rate of change of the absolute or relative fluorescence intensity over a predetermined time.

When the time rate of change of the absolute fluorescence intensity over a predetermined time is used as the fluorescence intensity increase rate, the fluorescence intensity increase rate may be obtained by calculating a time rate of change dFt/dt of Ft over a predetermined time. Here, Ft represents a fluorescence intensity of cells (measured value itself) at any stage after the sample is brought into contact and before the fluorescence intensity reaches a plateau. The predetermined time refers to a time between any two stages after the sample is brought into contact and before the fluorescence intensity reaches a plateau. dFt/dt may be obtained by calculating a slope of a curve obtained by plotting Ft against the time elapsed after the sample has been brought into contact. The maximum value of dFt/dt may be used as the fluorescence intensity increase rate.

As an example, when the fluorescence intensities of cells at stages after times t1 and t2 have elapsed after the sample has been brought into contact are represented by Ft1 and Ft2, respectively, the fluorescence intensity increase rate may be calculated using the following Formula (1).

$$\text{Fluorescence intensity increase rate} = (Ft2\text{-}Ft1)/(t2\text{-}t1) \cdots (1)$$

Note that the stage after time t1 has elapsed and the stage after the time t2 has elapsed after the sample has been brought into contact are both stages before the fluorescence intensity reaches a plateau, and t2>t1.

When the time rate of change of the relative fluorescence intensity over a predetermined time is used as the fluorescence intensity increase rate, the fluorescence intensity increase rate may be obtained by calculating a time rate of change d/dt(Ft/F0) of Ft/F0 over a predetermined time. Here, F0 represents the fluorescence intensity (measured value itself) of cells before the sample is brought into contact. The predetermined time refers to a time between any two stages after the sample is brought into contact and before the fluorescence intensity reaches a plateau. As an example, the fluorescence intensity increase rate may be calculated using the following Formula (2).

$$\text{Fluorescence intensity increase rate} = (Ft2\text{-}Ft1)/F0/(t2\text{-}t1) \cdots (2)$$

Alternatively, the fluorescence intensity increase rate may be obtained by calculating a time rate of change dIt/dt of It over a predetermined time. Here, It is (Ft-F0)/F0. The predetermined time represents a time between any two stages after the sample is brought into contact and before the fluorescence intensity reaches a plateau. dIt/dt may be obtained by calculating a slope of a curve obtained by plotting It against the time elapsed after the sample has been brought into contact. The maximum value of dIt/dt may be used as the fluorescence intensity increase rate. As an example, the fluorescence intensity increase rate may be calculated using the following Formula (3). When Formula (3) is modified, the same formula as Formula (2) is obtained.

$$\text{Fluorescence intensity increase rate} = (It2\text{-}It1)/(t2\text{-}t1) \cdots (3)$$

In step 1c, among all the cells, arbitrary cells exhibiting a fluorescence intensity increase rate within the top 50% are selected. Preferably, among all the cells, cells in which the maximum value of the fluorescence intensity increase rate is within the top 50%, 40%, 30%, 25%, or 20% are selected. The cells to be selected may be all the cells or some of the cells among the cells in which the fluorescence intensity increase rate is within the top 50%.

The selected cells may be first collected, and then used to detect the target substance in the sample or determine the concentration thereof. Alternatively, the selected cells may be used directly without collecting to detect the target substance in the sample or determine the concentration thereof. Since the cells selected by the method according to the present invention is highly responsive to the target substance, it is possible to detect the target substance in the sample with high sensitivity by using the cells according to the present invention.

A method for detecting a target substance in a sample or determining the concentration thereof using the selected and collected cells is not particularly limited, and known methods may be used. For example, the target substance in the sample may be detected or the concentration thereof may be determined, by culturing the collected cells by a known method and analyzing the fluorescence that is emitted when the sample is brought into contact with the cultured cells. However, in a case where the fluorescent indicator possessed by the selected cells is not genetically encoded, a fluorescent indicator such as a fluorescent dye needs to be present in the cultured cells. For the detection of the target substance and the determination of the concentration thereof, the fluorescence intensity increase rate may be used, or the fluorescence intensity at any stage before the fluorescence intensity reaches a plateau or at a stage when the fluorescence intensity reaches a plateau may be used.

The selected cells may also be used directly without collecting to detect the target substance in the sample. In that case, the presence of the target substance in the sample may be detected by bringing the sample that may contain a target substance into contact with at least the selected cells among all the cells and analyzing the fluorescence emitted from the selected cells.

The selected cells may be used directly without collecting to determine the concentration of the target substance in the sample. The present invention also provides a method for determining a concentration of a target substance with an unknown concentration in a sample, comprising steps of:

(2a) bringing a standard sample containing a target substance with a known concentration into contact with a plurality of cells:

(2b) calculating a fluorescence intensity increase rate for each of the cells;

(2c) repeating a combination of step 2a and step 2b using one or more standard samples containing the target substance with different concentrations to calculate a fluorescence intensity increase rate for each standard sample;

(2d) bringing a sample containing a target substance with an unknown concentration into contact with the cells,

(2e) calculating a fluorescence intensity increase rate for each of the cells after the sample is brought into contact;

(2f) selecting arbitrary cells in which the fluorescence intensity increase rate calculated for the sample or any of the standard samples is within the top 50% among the cells; and

(2g) comparing the fluorescence intensity increase rate calculated for the sample in step 2e with the fluorescence intensity increase rate calculated for the standard samples in steps 2b and 2c to calculate a concentration of the target substance in the sample. The increase rates compared in step 2g are the increase rates calculated for the cells selected in step 2f. In addition, steps 2a, 2b, 2c, 2d, 2e, and 2g are performed in this order, and step 2f is performed at any stage after step 2b and before step 2g.

Step 2a to step 2c relate to measurement of the fluorescence intensity increase rate of the cells when standard samples are used. Details of step 2a and step 2b are the same as described for step 1a and step 1b. However, in step 2a and step 2b, a standard sample containing a target substance with a known concentration is used instead of the sample. In step 2c, the concentration of the target substance is associated with the fluorescence intensity increase rate of each cell by repeating a combination of step 2a and step 2b, using one or more standard samples. A calibration curve showing the relationship between the concentration of the target substance and the fluorescence intensity increase rate of each cell may be created. The number of standard samples used is not particularly limited, and by using more standard samples, it is possible to create a more accurate calibration curve, and thus it is possible to determine the concentration of the target substance in the sample more accurately.

Step 2d and step 2e relate to measurement of the fluorescence intensity increase rate of the cells when the sample containing a target substance with an unknown concentration is used. Details of step 2d and step 2e are the same as described for step 1a and step 1b. However, in step 2d and step 2e, a sample containing a target substance with an unknown concentration is used.

Step 2f relates to selection of cells. Details of step 2f are the same as described for step 1c. However, in step 2f, cell selection is performed based on data of the fluorescence intensity increase rate obtained in any of step 2b, step 2c, and step 2e. In other words, in step 2f, cells in which the increase rate calculated in step 2b is within the top 50% among all the cells may be selected, cells in which the increase rate calculated in step 2c is within the top 50% among all the cells may be selected, or cells in which the increase rate calculated in step 2e is within the top 50% among all the cells may be selected. That is, this step may be performed before step 2c or before step 2d, as long as it is after step 2b.

In a case where cell selection (step 2f) is performed between step 2b and step 2c, measurement of the fluorescence intensity increase rate in step 2c and step 2e does not need to be performed for all cells, and performing the measurement for at least the cells selected in step 2f is sufficient. Therefore, in step 2c and step 2d, it is sufficient that the standard sample or the sample be brought into contact at least with the cells selected step 2f. Similarly, in a case where cell selection (step 2f) is performed between step 2c and step 2d, measurement of the fluorescence intensity increase rate in step 2e does not need to be performed for all cells, and performing the measurement for at least the cells selected in step 2f is sufficient. In addition, in step 2d, it is sufficient that the sample be brought into contact with at least the cells selected in step 2f.

Step 2g relates to determination of the concentration of the target substance in the sample. In this step, the value of the fluorescence intensity increase rate calculated for the sample containing a target substance with an unknown concentration (step 2e) is compared with the value of the fluorescence intensity increase rate calculated for the standard sample (step 2b and step 2c), using, for example, the calibration curve, to determine the concentration of the target substance in the sample.

According to the method for determining a concentration of a target substance in a sample according to the above embodiment, since the concentration is determined based on the fluorescence of cells that are highly responsive to the target substance, it is possible to detect the target substance in the sample with high sensitivity, and therefore, even if the concentration of the target substance is low, it is possible to determine the concentration thereof. In addition, according to the method for determining a concentration of a target substance in a sample according to the above embodiment, since the fluorescence intensity increase rate is used as an index for determining the concentration, it is possible to determine the concentration before the fluorescence intensity reaches a plateau. Therefore, compared to a conventional technique in which the concentration is determined based on the fluorescence intensity at the plateau, it is possible to determine the concentration of the target substance in the sample faster.

From the viewpoint of detecting the target substance with high sensitivity, step 2f of selecting cells having a high fluorescence intensity increase rate is important, but if the concentration of the target substance is higher than the detection limit, high sensitivity may not be required. Therefore, another aspect of the present invention provides a method for determining a concentration of a target substance with an unknown concentration in a sample, comprising steps of:

(3a) bringing a standard sample containing a target substance with a known concentration into contact with a plurality of cells;
(3b) calculating a fluorescence intensity increase rate for each of the cells;
(3c) repeating a combination of step 3a and step 3b using one or more standard samples containing the target substance with different concentrations to calculate a fluorescence intensity increase rate for each standard sample;
(3d) bringing a sample containing a target substance with an unknown concentration into contact with the cells;
(3e) calculating a fluorescence intensity increase rate for each of the cells after the sample is brought into contact; and
(3f) comparing the increase rate of the sample calculated in step 3e with the increase rate of the standard sample calculated in step 3b and step 3c to calculate a concentration of the target substance in the sample, in this order.

Details of steps 3a to 3f are the same as described for steps 2a to 2e and 2g. However, the increase rate compared in step 3f may be an increase rate calculated for all cells or may be an increase rate calculated for any portion of the cells. According to the method for determining a concentration of a target substance in a sample according to the above another embodiment, since the fluorescence intensity increase rate is used as an index for determining the concentration, it is possible to determine the concentration before the fluorescence intensity reaches a plateau. Therefore, compared to a conventional technique in which the concentration is determined based on the fluorescence intensity at the plateau, it is possible to determine the concentration of the target substance in the sample faster. In particular, when the concentration of the target substance in the sample is low, since it takes a long time for the fluorescence intensity to reach a plateau, this method, which allows for determination of the concentration before fluorescence intensity reaches a plateau, is useful.

In the method for selecting cells highly responsive to a target substance and the method for determining a concentration of a target substance with an unknown concentration in a sample according to the present invention, various devices may be used. For example, a sample containing a target substance may be introduced into the flow cell holding cells, and fluorescence emitted from the cells may be analyzed as described above. Alternatively, a device comprising a plurality of recesses in which a single cell can be captured may be used. After cells are captured in the recesses, a sample containing a target substance is introduced into the recesses, and fluorescence emitted from the cells may be analyzed as described above. In addition, a micro device comprising fine channels may also be used.

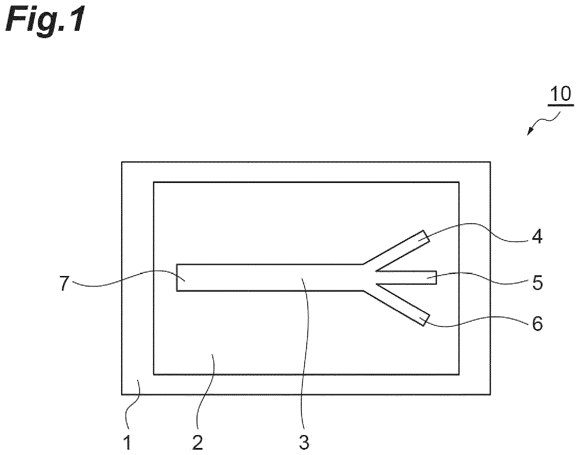

FIG. 1 shows an example of a microchannel device. A microchannel device 10 shown in FIG. 1 comprises a substrate 2 having a fork-shaped groove on one main surface, and a cover glass 1 laminated on the main surface on the side of the substrate 2 on which the groove is formed. The groove provided on the substrate 2 comprises a channel 3, three inlets 4, 5, and 6 provided at one end of the channel 3, and an outlet 7 provided at the other end of the channel 3, and has a fork shape. The substrate 2 is not particularly limited, and may be made of, for example, a resin such as silicone rubber (for example, dimethylpolysiloxane). When the substrate 2 is made of a resin, the channel 3, the inlets 4, 5, and 6, and the outlet 7 may be easily formed by photolithography. A syringe (not shown) filled with a cell suspension or a solution may be connected to the three inlets 4, 5, and 6. The cell suspension or solution introduced from the inlets 4, 5, and 6 into the channel 3 is discharged from the outlet 7 to the outside of the microchannel device 10. As necessary, the surface of the channel 3 may be modified with a cell adhesion substrate such as polylysine, or polyethylene glycol (PEG) phospholipid.

The microchannel device 10 may be used in the method for selecting cells highly responsive to a target substance, for example, as follows. First, the surface of the channel 3 is modified with a cell adhesion substrate. Syringes S4, S5, and S6 are connected to the inlets 4, 5, and 6, respectively. The syringes S4, S5, and S6 are filled with a cell suspension, a buffer, and a sample containing a target substance, respectively. The buffer may be, for example, a Ringer's solution (40 mM NaCl, 5.6 mM KCl, 4.5 mM $CaCl_2$, 11.26 mM $MgCl_2$, 10 mM HEPES, and 9.4 mM D-glucose, pH 7.2). Before step 1a, a cell suspension is introduced from the syringe S4 to the channel 3. The cells introduced into the channel 3 adhere to the channel 3 due to an action of the cell adhesion substrate. After the cells have adhered, introduction of the cell suspension from the syringe S4 is stopped. The buffer is introduced from the syringe S5 into the channel 3 to wash away cells that did not adhere to the channel 3. Introduction of the buffer from the syringe S5 is stopped, and the sample is introduced from the syringe S6 into the channel 3 to bring the sample into contact with the cells adhered to the channel 3 (step 1a). Fluorescence emitted from the cells is analyzed and highly responsive cells are selected by the above described method (steps 1b and 1c). As necessary, the selected cells may be collected from the channel 3 with a known device such as a pipette. Similarly, the microchannel device 10 may also be used in the method for determining a concentration of a target substance in a sample.

Alternatively, the microchannel device 10 may be used in the method for selecting cells highly responsive to a target substance, for example, as follows. First, the syringes S4, S5, and S6 are connected to the inlets 4, 5, and 6, respectively. The syringes S4 and S6 are filled with a sample containing a target substance, and the syringe S5 is filled with a cell suspension. The cell suspension or sample is introduced into the channel 3 from the syringes S4, S5, and S6 at the same time. By adjusting the pressure of the syringes S4, S5, and S6, a 3-layer laminar flow consisting of a layer of cell suspension layer and two layers of sample sandwiching the layer of cell suspension is formed in the channel 3. It is preferable to adjust the width of the cell suspension layer so that the cells are aligned in a single line within the layer. The target substance in the sample diffuses in the laminar flow, and the diffused target substance comes in contact with the cells in the cell suspension layer downstream of the channel 3 (step 1a). The fluorescence emitted from the cells is analyzed, and highly responsive cells are selected by the above method (steps 1b and 1c). Since the cells are discharged through the outlet 7 to the outside of the microchannel device 10 while keeping the single line, it is possible to collect only the selected cells from the discharged cells, as necessary. Similarly, the microchannel device 10 may also be used in the method for determining a concentration of a target substance in a sample.

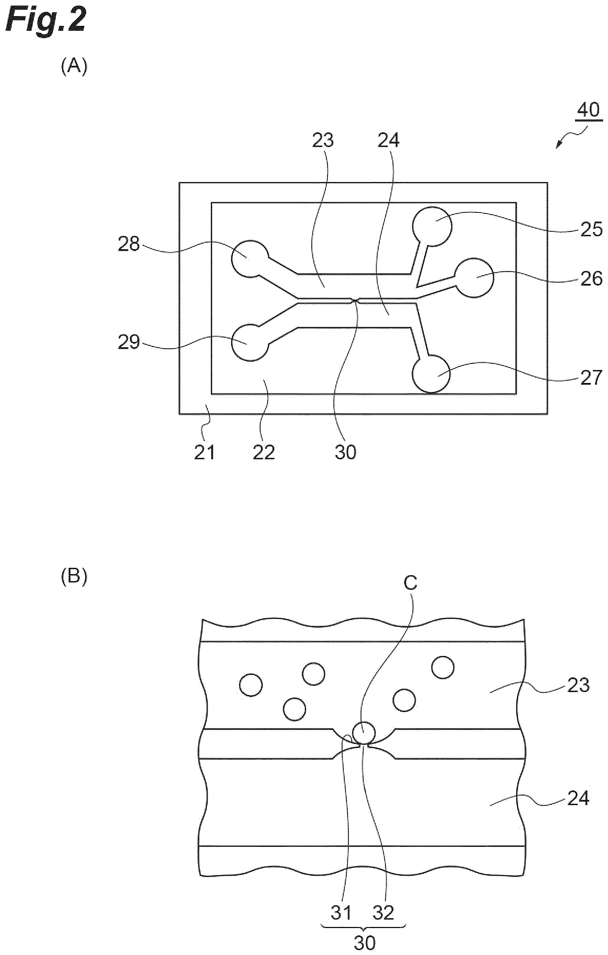

FIGS. 2(A) and 2(B) show another example of a microchannel device. A microchannel device 40 shown in FIG. 2(A) comprises a channel 23, a channel 24 adjacent to the channel 23, and a communicating portion 30 connecting the channel 23 to the channel 24. The channel 23, the channel 24, and the communicating portion 30 are all grooves provided in a substrate 22, and a cover glass 21 is laminated on the main surface on the side of the substrate 22 on which the groove is formed. The substrate 22 is not particularly limited, and may be made of, for example, a resin such as silicone rubber (for example, dimethylpolysiloxane). When the substrate 22 is made of a resin, the channel 23, the channel 24, and the communicating portion 30 may be easily formed by photolithography.

Inlets 25 and 26, and an outlet 28 for a liquid are provided in the channel 23, and an inlet 27 and an outlet 29 for a liquid are provided in the channel 24. A suspension containing cells C is injected into the inlet 25. Other solutions such as a sample, a standard sample, and a buffer are injected into the remaining inlets. The cell suspension or solution introduced from the inlets 25 and 26 into the channel 23 is discharged from the outlet 28 to the outside of the microchannel device 40, and the solution introduced from the inlet 27 into the channel 24 is discharged from the outlet 29 to the outside of the microchannel device 40. The suspension and the solution may be injected into inlets, for example, using a syringe. The number of inlets may be the same as the number of solutions used, but at least one inlet is sufficient for one channel. Therefore, the inlet 26 may not be provided, or one or more inlets may be added to the channel 24 and/or 23.

FIG. 2(B) shows an enlarged view of the communicating portion 30. The communicating portion 30 comprises a hole 32 connecting the channel 23 to the channel 24 and an opening (opening end) 31 in which cells C can be captured. Here, "cells C can be captured" means that the cells C present in the channel 23 can be held in the opening 31 on the side of the channel 23 under conditions in which the pressure in the channel 23 is higher than the pressure in the channel 24. In FIG. 2(B), the opening 31 forms a depression, but the shape of the opening 31 is not particularly limited as long as it can capture cells C. The communicating portion 30 needs to have a shape through which the cells C cannot pass. Therefore, it is preferable that the hole diameter of the hole 32 be sufficiently smaller than the diameter of the cell C. For example, when the cells are Sf21, the hole diameter of the hole 32 may be 1 $\mu$m to 15 $\mu$m. In addition, in FIGS. 2(A) and 2(B), the communicating portion 30 connects the channel 23 to the channel 24 via the hole 32, but the hole 32 may be replaced with a slit. The opening 31 needs only be provided on the side of the channel 23 in which the cells C are present, and it is not necessary to provide an opening on the side of the channel 24.

Next, some examples of a method for selecting cells highly responsive to a target substance using the microchannel device 40 will be described.

When the microchannel device 40 is used, step 1a may, for example, comprise steps of:

(A1) introducing a suspension containing a plurality of cells C into the channel 23 so that the pressure in the channel 23 is higher than the pressure in the channel 24 to capture the cells C in the opening 31 on the side of the channel 23; and

(A2) introducing a sample containing a target substance into the channel 23 while maintaining a pressure difference between the channel 23 and the channel 24 to bring the sample containing a target substance into contact with the captured cells C.

In step A1, the cell suspension may be introduced into the channel 23 via the inlet 25. While the suspension containing cells C flows through the channel 23, a buffer such as a Ringer's solution may be introduced into the channel 24 via the inlet 27. A method for generating a pressure difference between the channel 23 and the channel 24 is not particularly limited. For example, when the shapes of the channel 23 and the channel 24 are the same, the pressure in the channel 23 may be made higher than the pressure in the channel 24 by setting a driving pressure of the syringe introducing a cell suspension into the channel 23 higher than a driving pressure of the syringe introducing a buffer into the channel 24

In step A2, injection of the cell suspension may be stopped, and the sample may be introduced into the channel 23 via the inlet 26. At this point, a buffer may continue to flow into the channel 24. A pressure difference between the channel 23 and the channel 24 may be kept constant by adjusting the driving pressure of the syringes. The fluorescence emitted when the cells C captured in the opening 31 are brought into contact with the sample is analyzed in step 1b as described above, and highly responsive cells are selected in the subsequent step 1c.

The cells selected in step 1c may be collected by a method including:

(1d) introducing a liquid into the channel 24 so that the pressure in the channel 24 is higher than the pressure in the channel 23 to release the captured cells C from the opening 31; and
(1e) collecting the selected arbitrary cells among the released cells C.

In step 1d, the liquid introduced into the channel 24 is not limited, and may be, for example, a buffer. A method for reversing a pressure difference between the channel 23 and the channel 24 is not particularly limited. For example, when the shapes of the channel 23 and the channel 24 are the same, the pressure in the channel 24 may be made higher than the pressure in the channel 23 by setting a driving pressure of the syringe introducing a sample into the channel 23 lower than a driving pressure of the syringe introducing a buffer into the channel 24. The cells C released by the reversal of the pressure difference may be collected from the outlet 28.

In the microchannel device 40 shown in FIG. 2(A), only one inlet is provided in the channel 24, but another inlet (hereinafter referred to as an inlet 27b) may be added to the channel 24. In that case, in step A2, the sample may be introduced into the channel 24, instead of the channel 23, via the inlet 27b. Also, a buffer may be introduced into the channel 23 via the inlet 26. In step 1d, the pressure in the channel 24 may be made higher than the pressure in the channel 23 by setting a driving pressure of the syringe introducing a buffer into the channel 23 lower than a driving pressure of the syringe introducing a sample into the channel 24.

In the above example, an example in which individual inlets are provided for each suspension and solution used has been described, but suspensions and a plurality of solutions may also be introduced into the channel 23 or 24 from a single inlet.

The microchannel device 40 may also be used in the method for determining a concentration of a target substance with an unknown concentration in a sample according to the present invention. When the microchannel device 40 is used, before step 2a, cells C are captured in the opening 31 on the side of the channel 23 by the same step as step A1. In addition, in step 2a, a standard sample is brought into contact with the captured cells C by the same method as in step A2. Furthermore, in step 2d, a sample containing a target substance with an unknown concentration is brought into contact with the captured cells C by the same method as in step A2.

## Examples

Two sheets of paper (25 mm×5 mm) coated with vacuum grease were placed on a slide glass, and a cover glass (18 mm×18 mm) was superimposed thereon. The sheets of paper were arranged parallel to each other with a space of 5 mm between them. The surface of the slide glass to which bovine serum albumin (BSA) was adsorbed was then modified with PEG phospholipid (BAM) which is a cell adhesion substrate. This device was used as a flow cell.

Sf21 cells having a BmOR-3 protein, GCaMP6s protein, and Orco protein were suspended in a culture medium and introduced in a flow cell. After the cells adhered to the surface of the slide glass, a buffer was introduced into the flow cell, and the culture medium was washed away together with non-adhered cells. A Ringer's solution was used as the buffer.

The flow cell was placed under a fluorescence microscope, and the fluorescence of 100 cells was observed. A buffer containing 30 nM BAL was introduced into the flow cell, and fluorescent images of the cells were observed and recorded for several minutes. For each cell, a fluorescence intensity F0 immediately before the BAL solution was introduced and a fluorescence intensity Ft after the BAL solution was introduced were obtained from the fluorescent images, and It represented by the following Formula was calculated and recorded over time.

$$It = (Ft - F0)/F0 \times 100 (\%)$$

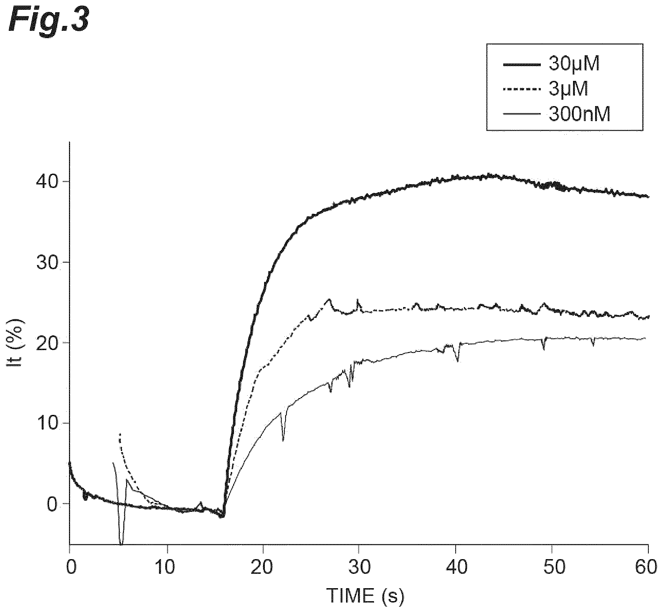

The same experiments were performed using 300 nM, 3000 nM, and 30000 nM BAL solutions. FIG. 3 is a graph showing change in It over time.

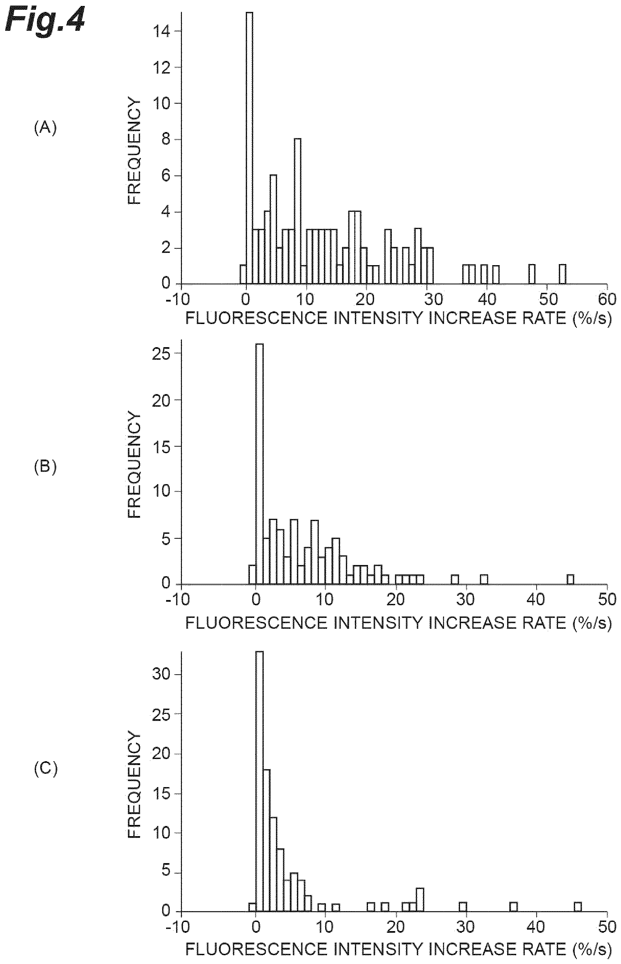

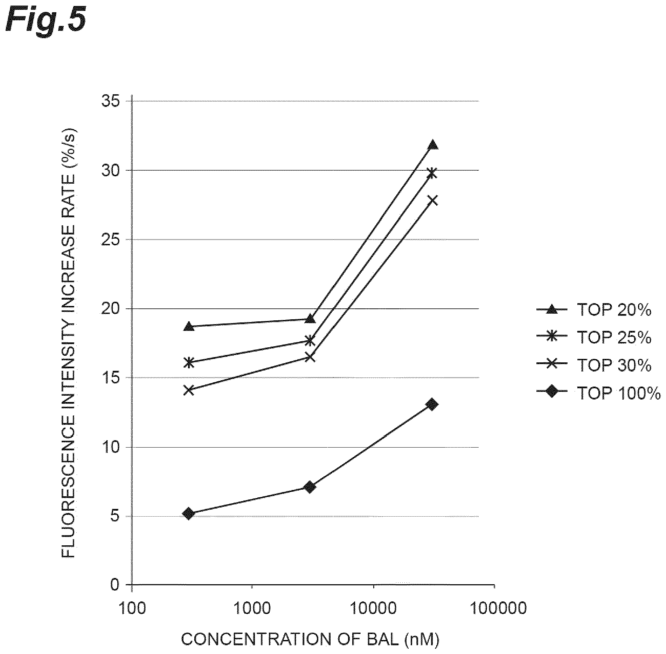

Linear fitting was repeated for the curve of It shown in FIG. 3 with a certain time width, and the change in the slope of the curve over time was determined. The slope of the curve represents the fluorescence intensity increase rate. The maximum value of the slope was determined for each cell, and histograms of the maximum value of the slope shown in FIGS. 4(A) to 4(C) were created. FIGS. 4(A), 4(B), and 4(C) show the results obtained when 30000 nM, 3000 nM, and 300 nM BAL solutions, respectively, were used. From the histograms, all values within the top 100% (that is, all cells), the top 30%, 25%, or 20% were selected for each concentration of the BAL solution, and average values thereof were calculated. The results are shown in FIG. 5.

**Reference Signs List**

1, 21 Cover glass
2, 22 Substrate
3, 23, 24 Channel
4, 5, 6, 25, 26, 27 Inlet
7, 28, 29 Outlet
10, 40 Microchannel device
30 Communicating portion
31 Opening
32 Hole
C Cell

**Claims**

1. A method for selecting cells highly responsive to a target substance, comprising steps of:

   (1a) bringing a sample containing a target substance into contact with a plurality of cells, wherein
   the cells are cells having a receptor for the target substance and a fluorescent indicator, and
   the fluorescent indicator emits fluorescence as a result of binding between the target substance and the receptor;
   (1b) calculating a fluorescence intensity increase rate for each of the cells; and
   (1c) selecting arbitrary cells exhibiting a fluorescence intensity increase rate within the top 50% among the plurality of cells.

2. The method according to claim 1, which is performed using a microchannel device comprising

   a first channel,
   a second channel adjacent to the first channel, and
   a communicating portion that connects the first channel to the second channel and has an opening on the side of the first channel in which the cells can be captured,
   wherein step 1a comprises:

      (A1) introducing a suspension containing the plurality of cells into the first channel so that a pressure in the first channel is higher than a pressure in the second channel to capture the cells in the opening on the side of the first channel; and
      (A2) introducing a sample containing a target substance into the first channel or the second channel while maintaining a pressure difference between the first channel and the second channel to bring the sample containing a target substance into contact with the captured cells, and

   wherein the method comprises, after step 1c, steps of:

      (1d) introducing a liquid into the second channel so that the pressure in the second channel is higher than the pressure in the first channel to release the captured cells from the opening; and
      (1e) collecting the selected arbitrary cells among the released cells.

3. The method according to claim 1 or 2, wherein the fluorescence intensity increase rate is a time rate of change of It over a predetermined time,

   wherein It is $(Ft-F0)/F0$,
   wherein F0 represents a fluorescence intensity of the cells before the sample is brought into contact,

Ft represents a fluorescence intensity of the cells at any stage after the sample is brought into contact and before the fluorescence intensity reaches a plateau, and
the predetermined time is a time between any two stages after the sample is brought into contact and before the fluorescence intensity reaches a plateau.

4.  The method according to any one of claims 1 to 3, wherein, in step 1c, arbitrary cells exhibiting a fluorescence intensity increase rate within the top 30% are selected among the plurality of cells.

5.  A method for determining a concentration of a target substance with an unknown concentration in a sample, comprising steps of:

    (2a) bringing a standard sample containing a target substance with a known concentration into contact with a plurality of cells, wherein

        the cells are cells having a receptor for the target substance and a fluorescent indicator, and
        the fluorescent indicator emits fluorescence as a result of binding between the target substance and the receptor;

    (2b) calculating a fluorescence intensity increase rate for each of the cells;
    (2c) repeating a combination of step 2a and step 2b using one or more standard samples containing the target substance with different concentrations to calculate a fluorescence intensity increase rate for each standard sample;
    (2d) bringing a sample containing a target substance with an unknown concentration into contact with the cells;
    (2e) calculating a fluorescence intensity increase rate for each of the cells after the sample is brought into contact;
    (2f) selecting arbitrary cells in which the fluorescence intensity increase rate calculated for the sample or any of the standard samples is within the top 50% among the cells; and
    (2g) comparing the fluorescence intensity increase rate calculated for the sample in step 2e with the fluorescence intensity increase rate calculated for the standard samples in step 2b and step 2c to calculate a concentration of the target substance in the sample, wherein the compared increase rates are the increase rates calculated for the cells selected in step 2f,
    wherein steps 2a, 2b, 2c, 2d, 2e, and 2g are performed in this order, and
    step 2f is performed at any stage after step 2b and before step 2g.

6.  The method according to claim 5, which is performed using a microchannel device comprising

    a first channel,
    a second channel adjacent to the first channel, and
    a communicating portion that connects the first channel to the second channel and has an opening on the side of the first channel in which the cells can be captured,
    wherein the method comprises, before step 2a, a step of introducing a suspension containing the plurality of cells into the first channel so that a pressure in the first channel is higher than a pressure in the second channel to capture the cells in the opening on the side of the first channel,
    step 2a comprises introducing the standard sample into the first channel or the second channel while maintaining a pressure difference between the first channel and the second channel to bring the standard sample into contact with the captured cells, and
    step 2d comprises introducing the sample into the first channel or the second channel while maintaining a pressure difference between the first channel and the second channel to bring the sample into contact with the captured cells.

7.  The method according to claim 5 or 6, wherein the fluorescence intensity increase rate is a time rate of change of It over a predetermined time,

    wherein It is (Ft-F0)/F0,
    wherein F0 represents a fluorescence intensity of the cells before the sample or the standard sample is brought into contact,
    Ft represents a fluorescence intensity of the cells at any stage after the sample or the standard sample is brought into contact and before the fluorescence intensity reaches a plateau, and
    the predetermined time is a time between any two stages after the sample or the standard sample is brought into contact and before the fluorescence intensity reaches a plateau.

8. The method according to any one of claims 5 to 7, wherein, in step 2f, arbitrary cells in which the fluorescence intensity increase rate calculated for the sample or any of the standard samples is within the top 30% are selected among the cells.

9. The method according to claim 3 or 7, wherein the fluorescence intensity increase rate is a maximum value of a time rate of change of It.

10. A method for determining a concentration of a target substance with an unknown concentration in a sample, comprising steps of:

(3a) bringing a standard sample containing a target substance with a known concentration into contact with a plurality of cells, wherein

the cells are cells having a receptor for the target substance and a fluorescent indicator, and
the fluorescent indicator emits fluorescence as a result of binding between the target substance and the receptor;

(3b) calculating a fluorescence intensity increase rate for each of the cells;
(3c) repeating a combination of step 3a and step 3b using one or more standard samples containing the target substance with different concentrations to calculate a fluorescence intensity increase rate for each standard sample;
(3d) bringing a sample containing a target substance with an unknown concentration into contact with the cells;
(3e) calculating a fluorescence intensity increase rate for each of the cells after the sample is brought into contact; and
(3f) comparing the fluorescence intensity increase rate calculated for the sample in step 3e with the fluorescence intensity increase rate calculated for the standard samples in step 3b and step 3c to calculate a concentration of the target substance in the sample, in this order.

11. The method according to any one of claims 1 to 10, wherein

the target substance is an odor substance, and
the plurality of cells are insect cells having an olfactory receptor of an insect and a calcium-sensitive fluorescent protein.

12. The method according to any one of claims 1 to 11, wherein

the target substance is bombykal, and
the plurality of cells are Sf21 cells having BmOR-3 protein and GCaMP6s protein.

**Fig.1**

# *Fig.2*

(A)

(B)

# Fig.3

*Fig.4*

(A)

(B)

(C)

## Fig.5

## INTERNATIONAL SEARCH REPORT

| International application No. |
| --- |
| PCT/JP2019/043560 |

| A. CLASSIFICATION OF SUBJECT MATTER |
| --- |
| Int.Cl. C12Q1/02(2006.01)i, C12M1/34(2006.01)i, G01N33/53(2006.01)i, G01N33/566(2006.01)i<br>FI: C12Q1/02, C12M1/34Z, G01N33/53Z, G01N33/566<br>According to International Patent Classification (IPC) or to both national classification and IPC |

| B. FIELDS SEARCHED |
| --- |
| Minimum documentation searched (classification system followed by classification symbols)<br>Int.Cl. C12Q1/00-3/00, C12M1/00-3/10, G01N33/48-33-98 |
| Documentation searched other than minimum documentation to the extent that such documents are included in the fields searched<br>    Published examined utility model applications of Japan     1922-1996<br>    Published unexamined utility model applications of Japan   1971-2020<br>    Registered utility model specifications of Japan           1996-2020<br>    Published registered utility model applications of Japan   1994-2020 |
| Electronic data base consulted during the international search (name of data base and, where practicable, search terms used)<br>    JSTPlus/JMEDPlus/JST7580(JDreamIII), MEDLINE/EMBASE/BIOSIS/WPIDS(STN) |

### C. DOCUMENTS CONSIDERED TO BE RELEVANT

| Category* | Citation of document, with indication, where appropriate, of the relevant passages | Relevant to claim No. |
| --- | --- | --- |
| Y | JP 2013-27376 A (KANZAKI, Ryohei) 07.02.2013 (2013-02-07), claims, paragraphs [0042]-[0047], fig. 5, 6, table 1 | 1-12 |
| Y | WO 03/100057 A1 (NATIONAL INSTITUTE OF ADVANCED INDUSTRIAL SCIENCE & TECHNOLOGY) 04.12.2003 (2003-12-04), page 1, lines 6-8, page 95, line 7 to page 96, line 23 | 1-12 |
| Y | JP 2008-136475 A (WASEDA UNIVERSITY) 19.06.2008 (2008-06-19), claims, paragraphs [0030], [0031], examples 2, 3, fig. 3, 4 | 1-12 |
| A | MITSUNO, Hidefumi et al., Novel cell-based odorant sensor elements based on insect odorant receptors, Biosens. Bioelectron., 2015, vol. 65, pp. 287-294, entire text | 1-12 |
| A | TERMTANASOMBAT, M. et al., Cell-based odorant sensor array for odor discrimination based on insect odorant receptors, J. Chem. Ecol., 2016, vol. 42, pp. 716-724, entire text | 1-12 |

☐ Further documents are listed in the continuation of Box C.    ☒ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search<br>    27.01.2020 | Date of mailing of the international search report<br>    04.02.2020 |
| --- | --- |
| Name and mailing address of the ISA/<br>    Japan Patent Office<br>    3-4-3, Kasumigaseki, Chiyoda-ku,<br>    Tokyo 100-8915, Japan | Authorized officer<br><br>Telephone No. |

Form PCT/ISA/210 (second sheet) (January 2015)

<table>
<tr><td colspan="2">**INTERNATIONAL SEARCH REPORT**<br>Information on patent family members</td><td>International application No.<br><br>PCT/JP2019/043560</td></tr>
</table>

```
JP 2013-27376 A      07.02.2013    (Family: none)

WO 03/100057 A1      04.12.2003    US 2007/0054266 A1
                                   paragraphs [0001], [0570],
                                   [0571]
                                   AU 2003241871 A

JP 2008-136475 A     19.06.2008    (Family: none)
```

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2013027376 A